# EUROPEAN PATENT SPECIFICATION

(11) **EP 3 913 064 B1**
(45) Date of publication and mention of the grant of the patent: **26.08.2026**
(21) Application number: 19910206.2
(22) Date of filing: 15.11.2019
(51) Int. Cl.: C12Q 1/04, A61K 45/00, A61P 31/04, C12Q 1/68

(54) **PAN-RESISTANT K. PNEUMONIAE DETECTION METHOD, ANTIMICROBIAL DRUG SCREENING METHOD, AND RECORDING MEDIUM/DATABASE**
NACHWEISVERFAHREN FÜR PAN-RESISTENTE K. PNEUMONIAE, SCREENING-VERFAHREN FÜR ANTIMIKROBIELLEN WIRKSTOFF UND AUFZEICHNUNGSMEDIUM/DATENBANK
PROCÉDÉ DE DÉTECTION DE K. PNEUMONIAE PAN-RÉSISTANTE, PROCÉDÉ DE CRIBLAGE DE MÉDICAMENT ANTIMICROBIEN ET SUPPORT D'ENREGISTREMENT/BASE DE DONNÉES

(30) Priority: 17.01.2019 JP 2019006012
(43) Date of publication of application: 24.11.2021
(73) Proprietor: Teikyo University, Tokyo 173-8605 (JP)
(72) Inventor: NISHIDA Satoshi, Tokyo 173-8605 (JP); ONO Yasuo, Tokyo 173-8605 (JP)
(74) Representative: Dehns
(86) International application number: PCT/JP2019/044965
(87) International publication number: WO 2020/148990

(56) References cited:
- PRANITA D. TAMMA ET AL: "Applying Rapid Whole-Genome Sequencing To Predict Phenotypic Antimicrobial Susceptibility Testing Results among Carbapenem-Resistant Klebsiella pneumoniae Clinical Isolates", ANTIMICROBIAL AGENTS AND CHEMOTHERAPY, vol. 63, no. 1, 29 October 2018 (2018-10-29), US, XP055725387, ISSN: 0066-4804, DOI: 10.1128/AAC.01923-18
- CONLAN SEAN ET AL: "Single-molecule sequencing to track plasmid diversity of hospital-associated carbapenemase-producing Enterobacteriaceae", SCIENCE TRANSLATIONAL MEDICINE, vol. 6, no. 254, 17 September 2014 (2014-09-17), pages 254ra126 - 1, XP009186925, ISSN: 1946-6234, [retrieved on 20140917], DOI: 10.1126/SCITRANSLMED.3009845
- DE MAN TOM J. B. ET AL: "Genomic Analysis of a Pan-Resistant Isolate of Klebsiella pneumoniae , United States 2016", vol. 9, no. 2, 2 May 2018 (2018-05-02), US, XP055968519, ISSN: 2161-2129, Retrieved from the Internet <URL:https://journals.asm.org/doi/pdf/10.1128/mBio.00440-18> DOI: 10.1128/mBio.00440-18
- SONNEVEND ÁGNES ET AL: "Multihospital Occurrence of Pan-Resistant Klebsiella pneumoniae Sequence Type 147 with an IS Ecp1 -Directed bla OXA-181 Insertion in the mgrB Gene in the United Arab Emirates", vol. 61, no. 7, 1 July 2017 (2017-07-01), US, XP055968562, ISSN: 0066-4804, Retrieved from the Internet <URL:https://journals.asm.org/doi/pdf/10.1128/AAC.00418-17> DOI: 10.1128/AAC.00418-17
- TAMMA, PRANITA D. ET AL.: "Applying Rapid Whole-Genome Sequencing To Predict Phenotypic Antimicrobial Susceptibility Testing Results among Carbapenem-Resistant Klebsiella pneumoniae Clinical Isolates", ANTIMICROBIAL AGENTS AND CHEMOTHERAPY, vol. 63, no. 1, 21 December 2018 (2018-12-21), pages e01923 - 18, XP055725387
- LOMONACO, SARA ET AL.: "Resistome of carbapenem- and colistin-resistant Klebsiella pneumoniae clinical isolates", PLOS ONE, vol. 13, no. 6, 8 June 2018 (2018-06-08), pages e0198526, XP055725392
- DONG, NING ET AL.: "Tracking microevolution events among ST11 carbapenemase- producing hypervirulent Klebsiella pneumoniae outbreak strains", EMERGING MICROBES & INFECTIONS, vol. 7, 2018, XP055725395
- BARRAGAN-PRADA, HUGO: "Emergence and dissemination of colistin-resistant Klebsiella pneumoniae isolates expressing OXA-48 plus CTX-M-15 in patients not previously treated with colistin in a Spanish university hospital", DIAGNOSTIC MICROBIOLOGY AND INFECTIOUS DISEASE, vol. 93, 8 September 2018 (2018-09-08), pages 147 - 153, XP085576508
- MIAO, MINHUI: "Genetic Diversity of Carbapenem-Resistant Enterobacteriaceae (CRE) Clinical Isolates From a Tertiary Hospital in Eastern China", FRONTIERS IN MICROBIOLOGY, vol. 9, 15 January 2019 (2019-01-15), XP055725511

## Description

### Technical Field

The present invention relates to a detection method for pan-resistant *K. pneumoniae,* a screening method for antimicrobial agents, and recording media and database.

### Background Art

Multidrug-resistant (MDR) organisms such as carbapenemase- and extended-spectrum β- lactamase (ESBL)-producing organisms are highly prevalent in certain regions of the world (non-patent literature 1 and 2). In particular, *Klebsiella pneumoniae* carbapenemase (KPC)-producing Gram-negative organisms are an increasing problem worldwide (non-patent literature 3 and 4), and travelers from endemic areas are particularly at risk of being infected with MDR bacteria. Infection with carbapenem-resistant Enterobacteriaceae is a serious clinical problem because it is difficult to treat using conventional antimicrobial agents.

In Japan, less than 1% of hospital-associated Enterobacteriaceae infections are carbapenem-resistant (non-patent literature 6). Usually, resistance to carbapenems is mediated by imipenemase (IMP)-type metallo-β-lactamases or by overexpression of AmpC cephalosporinases in combination with porin mutations. Carbapenemases, including KPC, New Delhi metallo-β-lactamase (NDM), and oxacillinase-48 (OXA-48), are increasingly common, however their detection remains sporadic (non-patent literature 4, 7).

In Asia, KPC isolates are limited to East Asia, including Southeast Asia (non-patent literature 3 and 7). The first KPC-producing *K. pneumoniae* reported in Japan (in 2014) was isolated from a patient originally hospitalized in Brazil (non-patent literature 8). KPC-producing *K. pneumoniae* is often MDR and poses serious problems in terms of clinical treatment and infection control.

Furthermore, for example, ESKAPE (*Enterococcus faecium, Staphylococcus aureus, Klebsiella pneumoniae, Acinetobacter baumannii, Pseudomonas aeruginosa, Enterobacter* sp.) pathogens frequently acquire drug resistance. Depending on the number of drugs to which the organism is resistant, they are categorized as MDR, extensively drug-resistant (XDR), or pandrug-resistant (PDR), and pathogens have pandrug-resistant (PDR) are not susceptible to any available antimicrobial agents (non-patent literature 9, 10).
There are currently no reports on the genetic structure and systems of drug resistance, because whole-genome sequences of pathogens that have acquired PDR are rarely available.

### Citation List

### Non-Patent Literature

[NPL 1] Jacoby GA, Munoz-Price LS. The New β-Lactamases. N. Engl. J. Med. 2005; 352:380-391.
[NPL 2] Wellington EM, Boxall AB, Cross P, et al. The role of the natural environment in the emergence of antibiotic resistance in Gram-negative bacteria. Lancet Infect. Dis. 2013;13:155-165.
[NPL 3] Munoz-Price LS, Poirel L, Bonomo RA, et al. Clinical epidemiology of the global expansion of Klebsiella pneumoniae carbapenemases. Lancet Infect. Dis. 2013; 13:785-796.
[NPL 4] Nordmann P, Poirel L. The difficult-to-control spread of carbapenemase producers among Enterobacteriaceae worldwide. Clin. Microbiol. Infect. 2014; 20:821-830.
[NPL 5] van der Bij AK, Pitout JDD. The role of international travel in the worldwide spread of multiresistant Enterobacteriaceae. J. Antimicrob. Chemother. 2012; 67:2090-2100.
[NPL 6] Infectious Disease Surveillance Center NI of ID. Carbapenem-resistant Enterobacteriaceae Infection, Japan. Infect. Agents Surveill. Rep. 2014; 35:281.
[NPL 7] Lee C-R, Lee JH, Park KS, Kim YB, Jeong BC, Lee SH. Global Dissemination of Carbapenemase-Producing Klebsiella pneumoniae: Epidemiology, Genetic Context, Treatment Options, and Detection Methods. Front. Microbiol. 2016; 7:895.
[NPL 8] Saito R, Takahashi R, Sawabe E, et al. First report of KPC-2 Carbapenemase-producing Klebsiella pneumoniae in Japan. Antimicrob. Agents Chemother. 2014; 58:2961-3.
[NPL 9] Falagas ME, Bliziotis IA. Pandrug-resistant Gram-negative bacteria: the dawn of the post-antibiotic era? Int. J. Antimicrob. Agents 2007; 29:630-636.
[NPL 10] Magiorakos A-P, Srinivasan A, Carey RB, et al. Multidrug-resistant, extensively drug-resistant and pandrug-resistant bacteria: an international expert proposal for interim standard definitions for acquired resistance. Clin. Microbiol. Infect. 2012; 18:268-281.
[NPL 11] Jolley KA, Maiden MCJ. BIGSdb: Scalable analysis of bacterial genome variation at the population level. BMC Bioinformatics 2010; 11:595.
[NPL 12] Jia B, Raphenya AR, Alcock B, et al. CARD 2017: expansion and model-centric curation of the comprehensive antibiotic resistance database. Nucleic Acids Res. 2017; 45:D566-D573.
[NPL 13] Arndt D, Grant JR, Marcu A, et al. PHASTER: a better, faster version of the PHAST phage search tool. Nucleic Acids Res. 2016; 44:W16-W21.
[NPL 14] Sullivan MJ, Petty NK, Beatson SA. Easyfig: a genome comparison visualizer. Bioinformatics 2011; 27:1009-1010.
[NPL 15] Alikhan N-F, Petty NK, Ben Zakour NL, Beatson SA. BLAST Ring Image Generator (BRIG): simple prokaryote genome comparisons. BMC Genomics 2011; 12:402.
[NPL 16] Darling AE, Mau B, Perna NT. progressiveMauve: Multiple Genome Alignment with Gene Gain, Loss and Rearrangement. PLoS One 2010; 5:14
[NPL 17] Zhang Z, Schwartz S, Wagner L, Miller W. A Greedy Algorithm for Aligning DNA Sequences. J. Comput. Biol. 2000; 7:203-214.
[NPL 18] Xiang D-R, Li J-J, Sheng Z-K, et al. Complete Sequence of a Novel IncR-F33:A-:B- Plasmid, pKP1034, Harboring fosA3, blaKPC-2, blaCTX-M-65, blaSHV-12, and rmtB from an Epidemic Klebsiella pneumoniae Sequence Type 11 Strain in China. Antimicrob. Agents Chemother. 2015; 60:1343-8.
[NPL 19] Liu P, Li P, Jiang X, et al. Complete Genome Sequence of Klebsiella pneumoniae subsp. pneumoniae HS11286, a Multidrug-Resistant Strain Isolated from Human Sputum. J.Bacteriol. 2012; 194:1841-1842.
[NPL 20] Lam MMC, Wick RR, Wyres KL, et al. Genetic diversity, mobilisation and spread of the yersiniabactin-encoding mobile element ICEKp in Klebsiella pneumoniae populations. Microb. Genomics 2018;
[NPL 21] Blankschien MD, Potrykus K, Grace E, et al. TraR, a Homolog of a RNAP Secondary Channel Interactor, Modulates Transcription. PLoS Genet. 2009; 5:e1000345.
[NPL 22] Gopalkrishnan S, Ross W, Chen AY, Gourse RL. TraR directly regulates transcription initiation by mimicking the combined effects of the global regulators DksA and ppGpp. Proc. Natl. Acad. Sci. 2017; 114:E5539-E5548.
[NPL 23] Gu D, Dong N, Zheng Z, et al. A fatal outbreak of ST11 carbapenem-resistant hypervirulent Klebsiella pneumoniae in a Chinese hospital: a molecular epidemiological study. Lancet Infect. Dis. 2017;
[NPL 24] Huang Y-H, Chou S-H, Liang S-W, et al. Emergence of an XDR and carbapenemase-producing hypervirulent Klebsiella pneumoniae strain in Taiwan. J. Antimicrob. Chemother. 2018; 73:2039-2046.
[NPL 25] Shoma S, Kamruzzaman M, Ginn AN, Iredell JR, Partridge SR. Characterization of multidrug-resistant Klebsiella pneumoniae from Australia carrying blaNDM-1. Diagn. Microbiol. Infect. Dis. 2014; 78:93-97.
[NPL 26] Zhang Y, Jiang X, Wang Y, et al. Contribution of β-Lactamases and Porin Proteins OmpK35 and OmpK36 to Carbapenem Resistance in Clinical Isolates of KPC-2-Producing Klebsiella pneumoniae. Antimicrob. Agents Chemother. 2014; 58:1214-1217.
[NPL 27] Deguchi T, Fukuoka A, Yasuda M, Nakano M, Ozeki S, Kanematsu E, et al. Alterations in the GyrA subunit of DNA gyrase and the ParC subunit of topoisomerase IV in quinolone-resistant clinical isolates of Klebsiella pneumoniae. Antimicrob Agents Chemother 1997;41:699-701.
[NPL 28] Sheng Z-K, Hu F, Wang W, Guo Q, Chen Z, Xu X, et al. Mechanisms of Tigecycline Resistance among Klebsiella pneumoniae Clinical Isolates. Antimicrob Agents Chemother 2014;58:6982-5. doi:10.1128/AAC.03808-14.

### Summary of Invention

### Technical Problem

The inventors obtained a novel PDR *K. pneumoniae* ST11 isolate as one of the three distinct carbapenem-resistant Gram-negative bacteria from a patient transferred from a hospital in Jakarta, Indonesia, and performed a complete genome analysis of this strain.

The present invention was accomplished in consideration of the aforementioned situation, and an object thereof is to provide a detection method for pan-resistant *K*. *pneumoniae,* a screening method for antimicrobial agents, and recording media and database by using a novel PDR *K. pneumoniae* and sequence information from this complete genome analysis.

### Solution to Problem

The present invention is characterized by the following.

A detection method for pan-resistant *K. pneumoniae* comprising of:
performing complete genome analysis of the bacteria contained in the sample; and
comparing the nucleotide sequence contained in the obtained complete genome with nucleotide sequences of SEQ ID NO: 1 and 2, and determining whether or not the bacteria in the sample is pan-resistant *K. pneumoniae* based on the rate of agreement, wherein when the rate of agreement is 99.9% or higher the bacteria in the sample is pan-resistant *K. pneumoniae.*

A screening method for antimicrobial agents comprising of:
supplying a sample containing a candidate antimicrobial agent to pan-resistant *K*. *pneumoniae* which has chromosomal DNA comprising of the nucleotide sequence of SEQ ID NO: 1 and plasmid DNA comprising of the nucleotide sequence of SEQ ID NO: 2.

A recording media and database characterized by the storage of nucleotide sequence information of SEQ ID NO: 1 and 2.

### Advantageous Effects of Invention

According to the detection method for pan-resistant *K. pneumoniae* of the present invention, it is possible to accurately determine whether the bacteria in a sample are pan-resistant *K. pneumoniae* or not. According to the screening method for antimicrobial agents of the present invention, antimicrobial agents are effective against not only pan-resistant *K. pneumoniae* but also other bacteria can be reliably screened. According to recording media and database of the present invention enables the use of the sequence information of pan-resistant *K. pneumoniae* for various types of analysis, research, etc. and commercial use.

### Brief Description of Drawings

[Fig.1] This figure shows the phylogenetic tree of *K. pneumoniae* chromosomes. The phylogenetic tree of the complete *K. pneumoniae* chromosome sequence constructed from the complete genome is shown with single nucleotide polymorphisms using PathoBacTyper.
[Fig.2] This figure shows the phylogenetic analysis of *K. pneumoniae* ST11 isolates. (a) Phylogenetic tree of 48 *K. pneumoniae* ST11 isolates constructed by complete genome single nucleotide polymorphism analysis using PathoBacTyper. Black circles indicate strains reported to harbor virulence plasmids. (b) Comparative analysis of the plasmids possessed by *K. pneumoniae* ST11 isolates. Easyfig is used to compare the plasmid sequences. The top, middle, and bottom sequences represent pKP1034, p69-2, and pTK1401.
[Fig.3] This figure shows the comparative genomic analysis of *K. pneumoniae* ST11 isolates KP69 and TK1401. Chromosomal sequences of KP69 and TK1401 are compared using BLAST Ring Image Generator (BRIG).
[Fig.4] This figure shows the comparative genomic analysis of *K. pneumoniae* ST11 isolates KP69 and TK1401. The plasmid sequences of KP69 and TK1401 are compared using BRIG.
[Fig.5] This figure shows the comparative analysis of the genomes of *K. pneumoniae* ST11 isolates. (a) shows the comparative analysis of the chromosomes of *K. pneumoniae* ST11 isolates JM45, HS11286, KP69, and TK1401 (performed using ProgressiveMauve). The locations of the prophage and ItrA (group II intron reverse transcriptase) genes are indicated by arrows. (b) shows the comparative analysis of plasmids possessed by *K. pneumoniae* ST11 isolates. Easyfig was used to compare the plasmid sequences. The top, middle, and bottom sequences represent pKP1034, pTK1401, and pKSH203-KPC.
[Fig.6] This figure shows the phylogenetic tree of the complete *K. pneumoniae* plasmid sequence. parsnp is used to show single nucleotide polymorphisms.

### Description of Embodiments

The inventors have conducted a whole-genome analysis of *K. pneumoniae* isolated from Japanese patients with a history of treatment in Indonesia and determined the nucleotide sequence of 5.5 Mb of chromosomal DNA (Sequence Listing: SEQ ID NO: 1) and 128 kb of plasmid DNA (Sequence Listing: SEQ ID NO: 2).

This *K. pneumoniae* has 16 drug resistance genes to 9 classes of antimicrobial agents, and has been confirmed to be pan-resistant (PDR), in which commercially available antimicrobial agents are ineffective.

An embodiment of the present invention of the method for detecting pan-resistant *K. pneumoniae* will be explained as follows.

A detection method for pan-resistant *K. pneumoniae* of the present invention comprising the following steps of:
(1) performing complete genome analysis of the bacteria contained in the sample; and
(2) comparing the nucleotide sequence contained in the obtained complete genome with nucleotide sequences of SEQ ID NO: 1 and 2, and determining whether or not the bacteria in the sample is pan-resistant *K. pneumoniae* based on the rate of agreement, wherein when the rate of agreement is 99.9% or higher the bacteria in the sample is pan-resistant *K. pneumoniae.*

In step (1), for example, genomic DNA is isolated from a sample collected from a patient or the environment using known methods, and the genome of the bacteria is analyzed using known methods (e.g., MiSeq, Genome Analyzer series, HiSeq series from Illumina, ion proton, ion PGM, SoLid series from Life technologies, GS series from Roche, PacBio RS series from PacBio, etc.). This step provides sequence information on the complete genome of the bacteria contained in the sample.

In step (2), the nucleotide sequence of the complete genome obtained in step (1) above is compared with the nucleotide sequence of SEQ ID NO: 1 and 2, and whether the bacteria in the sample is pan-resistant *K. pneumoniae* or not is determined based on the rate of agreement.

The complete genome sequence information entered into the computer can be compared with the sequence of SEQ ID NO: 1 and 2 by processing commercially available software (e.g., calculating the rate of agreement). If the rate of agreement of the nucleotide sequence is 99.9% or higher, it can be determined that the bacteria in the sample is pan-resistant *K. pneumoniae.* In addition, the 16 drug resistance genes possessed by pan-resistant *K. pneumoniae* found by the inventors can be focused on in the determination.

In addition, the present invention also provides a recording media or database in which the sequence information of SEQ ID NO: 1 and 2 is stored. The recording media is not particularly limited, but examples may include magnetic storage media, such as floppy disks, hard disk storage media and magnetic tapes; optical storage media, such as CD-ROMs; and electrical storage media, such as RAM and ROM. The database can also be exemplified in the form of a computer-based system that has sequence information of SEQ ID NO: 1 and 2 stored in data storage means.

Next, the screening method for antimicrobial agents of the present invention will be explained.

A screening method for antimicrobial agents of the present invention comprises of supplying a sample containing a candidate antimicrobial agent to pan-resistant *K*. *pneumoniae* which has chromosomal DNA comprising of the nucleotide sequence of SEQ ID NO: 1 and plasmid DNA comprising of the nucleotide sequence of SEQ ID NO: 2.

The pan-resistant *K. pneumoniae* found by the inventors has chromosomal DNA consisting of the SEQ ID NO: 1 and plasmid DNA consisting of the SEQ ID NO: 2, and contains 16 drug resistance genes to 9 classes of antimicrobial agents. Therefore, the commercially available antimicrobial agents are ineffective against this pan-resistant *K*. *pneumoniae.* Therefore, samples containing candidate antimicrobial agents that are expected to be effective against pan-resistant *K. pneumoniae* are given to pan-resistant *K*. *pneumoniae.* If, for example, a decrease in the number of surviving pan-resistant *K*. *pneumoniae* can be confirmed, it can be evaluated as an antimicrobial agent effective not only against pan-resistant *K. pneumoniae* but also against other bacterium. In this way, the pan-resistant *K. pneumoniae* found by the inventors can be used to screen for new active ingredients for antimicrobial agents.

Furthermore, the sequence information obtained from this genome analysis of *K. pneumoniae* (SEQ ID NO: 1 and 2) or representative fragments thereof can be used for various analyses, studies, etc., by those skilled in the art.

For example, primers and probes used to detect pan-resistant *K. pneumoniae* found by the present inventors can be designed based on the sequence information shown in SEQ ID NO: 1 and 2. For example, the sequence information shown in SEQ ID NO: 1 and 2 can be used to establish methods for the specific detection of genomic DNA, such as PCR (Polymerase Chain Reaction) using primer sets, Southern hybridization method using probes, and FISH (Fluorescent in situ hybridization) method.

The method for detecting pan-resistant *K. pneumoniae,* the screening method for antimicrobial agents, and the recording medium and database are not limited to the above embodiments.

### Example

The invention is described in more detail by means of examples below, but the invention is not limited in any way to these examples.

### <1> Acquisition of the isolate

The *K. pneumoniae* isolate TK1401 was previously identified from fecal samples, tracheal samples (including aspirate and sputum), and pharyngeal and wound swab from a patient in a laboratory stool and swab surveillance-screening program for infection control. The patient was a 74-year-old Japanese man with a history of hospitalization at a hospital in Jakarta, Indonesia.

### <2>Culture and isolation of bacterial strains

The screening samples were cultured on CHROM Agar mSuperCARBA (Kanto Chemical, Tokyo, Japan) and stored in glycerol at -80°C. Frozen stocks of isolates were streaked on Lysogeny broth (LB) (Miller) agar (Becton Dickinson, Franklin Lakes, NJ, USA) and incubated at 37°C. A single colony of an isolate was grown at 37°C in LB (Miller) (Sigma-Aldrich, St Louis, MO, USA) prior to liquid culture in a Bio-shaker BR-40LF (Taitec, Tokyo, Japan). Genomic DNA was isolated from cells of *K. pneumoniae* TK1401 using the DNeasy Blood & Tissue Kit (QIAGEN, Hilden, Germany).

### <3>Antimicrobial susceptibility testing

Testing was performed using a MicroScan WalkAway apparatus (Beckman Coulter, Brea, CA, USA). The minimal inhibitory concentration (MIC) of colistin was confirmed using the Etest (bioMérieux, Marcy l'Etoile, France) and the broth microdilution method. Antimicrobial susceptibility was defined according to breakpoints listed in the Clinical and Laboratory Standards Institute (CLSI) guidelines (M100-S24).

### <4>Genome analysis

Genomic DNA was isolated from cells of *K. pneumoniae* TK1401 using the DNeasy Blood & Tissue Kit (QIAGEN, Hilden, Germany). The complete genome sequence was determined by single-molecule real-time sequencing (SMRT) using PacBio RSII (Pacific Biosciences, Menlo Park, CA, USA). A 1,896 Mb sequence was obtained, with 210,166 reads and an *N*₅₀ read length of 12,144 bp. Hierarchical Genome Assembly Process version 3 was used for the assembly of two circular contigs. The complete chromosome and plasmid sequences comprised 5,488,304 bp with 57.4% GC content, and 128,638 bp with 54.4% GC content, respectively (SEQ ID NO: 1 and 2). DDBJ Fast Annotation and Submission Tool beta identified 173 protein-coding regions in the plasmid and 5,156 protein-coding regions in the chromosome, with 9 copies of 5S, 8 copies of 16S, and 23S rRNA, and 86 tRNAs.

Genomic sequences were analyzed using the Bacterial Isolate Genome Sequence Database (BIGSdb; http://bigsdb.pasteur.fr/klebsiella/) (NPL 11), the Comprehensive Antibiotic Resistance Database (CARD; http://arpcard.mcmaster.ca) (NPL 12), and the Phage Search Tool Enhanced Release (PHASTER; http://phaster.ca) (NPL 13), and were compared using Easyfig v2.2.2 (NPL 14), the BLAST Ring Image Generator (BRIG) (NPL 15), and progressive Mauve (NPL 16). Genome similarity was analyzed using NCBI BLASTN 2.10.0 (NPL 17). Phylogenetic analysis was performed with whole-genome single nucleotide polymorphism (wgSNP) analysis using PathoBacTyper (http://hast.nhri.org.tw/PathoBacTyper/) and Harvest v1.1.2. A phylogenetic tree was drawn using FigTree v1.4.3. Genomes sequences were deposited in DDBJ with the BioProject number PRJDB8036.

### <5> Results

### (Antimicrobial susceptibility test)

The results of the antimicrobial susceptibility test are shown in Table 1.

**[Table 1]**

| Antimicrobial agent^{a} | *K. pneumoniae* TK1401 | Resistance genes^{c} |
|---|---|---|
| Penicillins | | |
| Ampicillin | **> 16** | *bla*_{TEM-1B}, *bfa*_{SHV-12,} ***bla*_{SHV-11},** *bla*_{CTX-M-65,} *bla*_{KPC-2} |
| Piperacillin | **> 64** | *bla*_{TEM-1B}, *bla*_{SHV-12}, ***bla*_{SHV-11},** *bla*_{CTX-M-65}, *bla*_{KPC-2} |
| Antoxicillin | **> 16** | *bla*_{TEM-1B}, *bla*_{SHV-12}, ***bla*_{SHV-11},** *bla*_{CTX-M*-*65}, *bla*_{KPC-2} |
| Cephalosporins | | |
| Cefazolin | **> 16** | *bla*_{TEM-1B}, *bla*_{SHV-12}, ***bla*_{SHV-11},** *bla*_{CTX-M-65}, *bla*_{KPC-2} |
| Cefotiam | **> 16** | *bla*_{TEM-1B}, *bla*_{SHV-12}, ***bla*_{SHV-11},** *blac*_{TX-M-65}, *bla*_{KPC-2} |
| Cefotaxim | **> 2** | *bla*_{CTX-M-65}, *bla*_{KPC-2} |
| Ceftazidime | **> 16** | *bla*_{CTX-M-65}*, bla*_{KPC-2} |
| Ceftriaxone | **> 2** | *bla*_{CTX-M-65}, *bla*_{KPC-2} |
| Cefepime | **> 16** | *bla*_{CTX-M-65} |
| Cefozopran | **> 16** | *bla*_{KPC-2} |
| Cefinetazole | **> 32** | *bla*_{KPC-2} |
| Cefaclor | **> 16** | *bla*_{KPC-2} |
| Cefdinir | **> 2** | *bla*_{KPC-2} |
| Cefpodoxime | **> 4** | *bla*_{KPC-2} |
| Flomoxef | **> 32** | *bla*_{KPC-2} |
| Carbapenems | | |
| Doripenem | **> 8** | *bla*_{KPC-2}, ***ompK36* variant** |
| Imipenem | **> 8** | *bla*_{KPC-2}, ***ompK36* variant** |
| Meropenem | **> 8** | *bla*_{KPC-2}, ***ompK36* variant** |
| Monobactains | | |
| Aztreonam | **>16** | *bla*_{CTX-M-65} |
| Penams | | |
| Sulbactan1/cephoperazon | **> 32** | *bla*_{KPC-2} |
| Tazobactam/piperacillin | **> 64** | *bla*_{KPC-2} |
| Fluoroquinolones | | |
| Ciprofloxacin | **> 2** | ***oqxAB*, *gyrA*D87G, *perC*S80I*, ram*RT162I, *ompK35* FS_aa29** |
| Levofloxacin | **> 4** | ***oqxAB*, *gyrA*D87G, *perC*S80I, *ram*RT162I*, ompK35* FS_aa29** |
| Aminoglycosides | | |
| Gentamicin | **> 8** | ***aadA2,** rmtB* |
| Amikacin | **> 32** | ***aadA2,** rmtB* |
| Tobramycin | **> 8** | ***aadA2,** rmtB* |
| Arbekacin^{b} | **> 256** | |
| Tetracyclines | | |
| Minocycline^{b} | **256** | ***ramR*T162I, *ompK35* FS_aa29** |
| Tigecycline^{b} | **16** | ***ramR*T162I** |
| Polymyxins | | |
| Colistin^{b} | **64** | ***mgrB* FS_aa8** |
| Polymyxin B^{b} | **64** | ***mgrB* FS_aa8** |
| Other | | |
| Trimethoprin/sulfamethoxazole | **> 2** | ***sul1*** |
| Fosfomycin^{b} | **> 256** | ***fosA*,*fosA3*** |
| Chloramphenicol^{b} | **> 256** | ***ramR*T162I, *ompK35* FS_aa29.** *catA2* |

| | | |
|---|---|---|
| a, Minimum inhibitory concentration (mg/L) values in bold represent resistant or non-susceptible values. b, Broth microdilution method. c, Resistance genes in bold are chromosomal genes. | | |

The isolate was not susceptible to penicillins, cephalosporins, carbapenems, monobactams, aminoglycosides, fluoroquinolones, polymyxins, tetracyclines, glycylcyclines, phenicols, trimethoprim-sulfamethoxazole, or fosfomycin. These data suggest that TK1401 had a PDR phenotype. This is thought to be the first report of a patient with PDR (pan-resistant) *K. pneumoniae* in Japan.

Genome sequencing of the *K. pneumoniae* isolate TK1401 revealed a 129 kb plasmid pTK1401 and a 5.5 Mb chromosome, which belonged to the genomic MLST (gMLST) ST11 group. The plasmid incompatibility group was a hybrid incompatibility group, IncR-F33: A-:B-. This compatibility group plasmid is a non-conjugative plasmid (NPL 18). In addition, no junction between *K. pneumoniae* isolate TK1401 and E. coli J53 was observed.

### (Genome analysis)

The complete genome sequence of *K. pneumoniae* isolate TK1401 is shown in SEQ ID NO 1 and 2. Specifically, chromosomal DNA is shown in SEQ ID NO: 1 in the sequence listing, and plasmid DNA is shown in SEQ ID NO: 2 in the sequence listing.

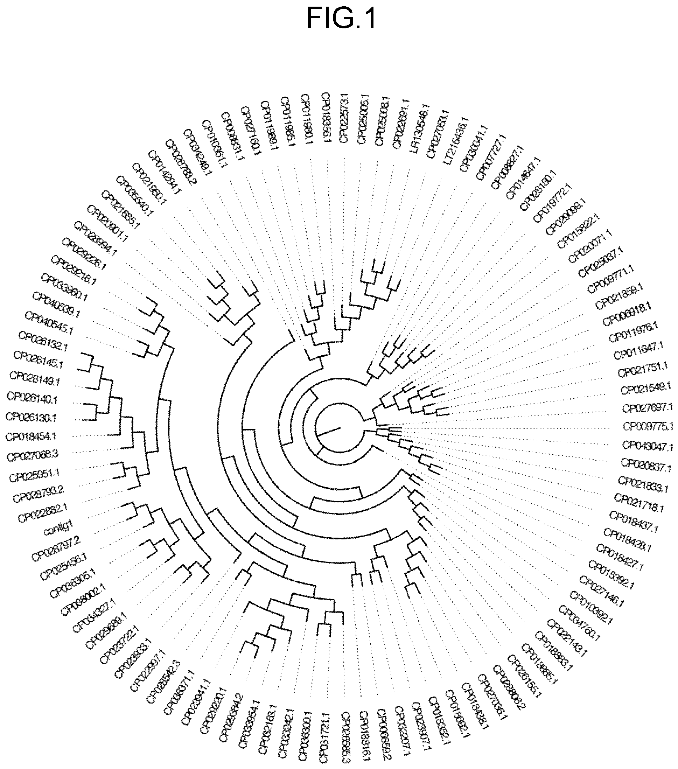

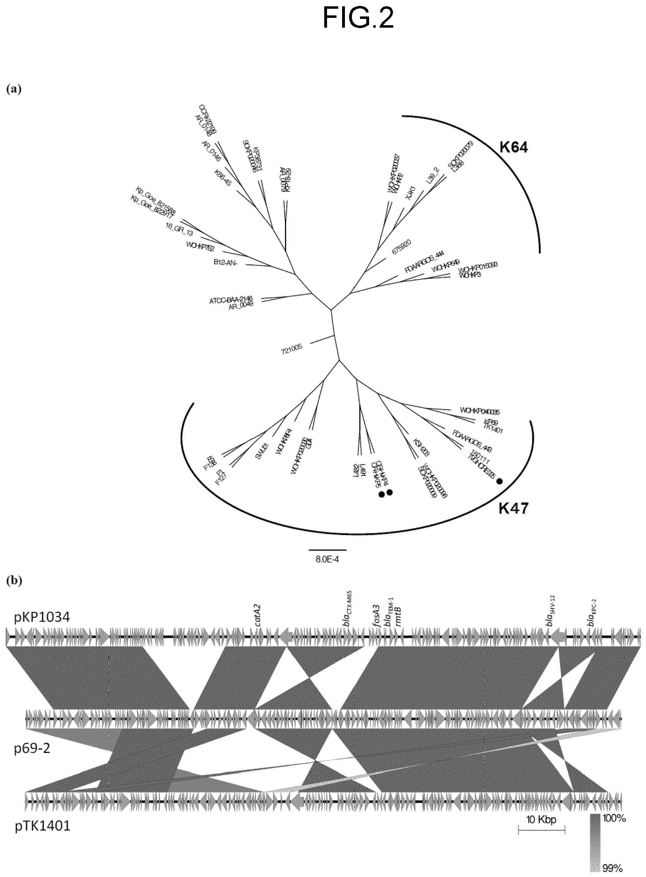

The TK1401 chromosome was grouped with the top 100 hits for *K. pneumoniae* isolates, with a single nucleotide polymorphism (SNP) ranging from 38 to 4,351. It clustered with ST11 isolates identified in Asia (Figure 1). Figure 2(a) shows a phylogenetic tree constructed from wgSNP compared the genome sequence of *K. pneumoniae* ST11 strain TK1401 with that of other *K. pneumoniae* ST11 strains mainly from China. Genomic capsular typing using *wzc* and *wzi* sequences in BIGSdb revealed that *K. pneumoniae* ST11 isolates mainly clustered with K47-type (*wzc47, wzi209*), K64-type (*wzc64, wzi64*), and other capsule synthesis (cps) genes. The TK1401 chromosome was most similar to that of KPC-2-producing *K. pneumoniae* isolate KP69 (GenBank accession number CP025456) isolated at Huashan Hospital, Shanghai Medical College, Fudan University in Shanghai, China (99% query coverage, 99% identity). Genome analysis of KP69 using CARD revealed that KP69 is not a pan-resistant genotype (Table 2). Tigecycline and polymyxins resistance mutations were identified in the *ramR* and *mgrB* genes of TK1401, but not in those of KP69.

**[Table 2]**

| Antibiotic class | TK1401 | | KP69 | | HS11286 | | JM45 | |
|---|---|---|---|---|---|---|---|---|
| | Chromosome | Plasmid | Chromosome | Plasmid | Chromosome | Plasmid | Chromosome | Plasmid |
| β-lactam | - | *bla*_{TEM-1} | - | *bla*_{TEM-1} | - | *bla*_{TEM-1} | - | *bla*_{TEM-1} |
| | *bla*_{SHV*-*11} | *bla*_{SHV-66} | *bla*_{SHV-11} | *bla*_{SHV-66} | *bla*_{SHV-11} | | *bla*_{SHV-11} | *bla*_{*v*VEB-3} |
| | - | *bla*_{CTX-M-65} | - | *bla*_{CTX-M-65} | - | *bla*_{CTX-M-14} | - | *bla*_{CTX-M-24} |
| Carbapenem | *ompK37* | *bla*_{KPC-2} | *ompK37* | *bla*_{KPC-1} | *ompK37* | *bla*_{KPC-2} | *ompK37* | *bla*_{KPC-2} |
| Fluoroquinolone | *oqxAB* | - | *oqxAB* | - | - | - | *oqxAB* | *-* |
| | *gyrA* D87G | - | *gyrA* D87G | - | *gyrA* S83I | - | *gyrA* S83I | - |
| | *parC* S80I | - | *parC* S80I | - | *parC* S80I | - | *parC* S80I | - |
| | *ompK36v2* | - | *ompK36v2* | - | *ompK36v1* | - | *ompK36v1* | *-* |
| Aminoglycoside | *aadA2* | *mtB* | *aadA2* | *rmtB* | - | *aadA2* | - | *aadA2* |
| | - | - | - | - | - | *AAC(3)-IIc* | - | *APH(3')-Ia* |
| | - | - | - | - | - | *APH(3")-Ib* | - | *rmtB* |
| | - | - | - | - | *-* | *APH(6)-Id* | *-* | - |
| | - | - | - | - | - | *rmtB* | - | - |
| Fosfomycin | *fosA6* | *fosA3* | *fosA6* | - | *fosA6* | - | *fosA6* | - |
| | *uhpT* E350Q | - | *uhpT* E350Q | - | *uhpT* E350Q | - | *uhpT* E350Q | |
| Terracycline (Glycylcycline) | *ramR* T162I | - | - | - | - | - | - | *tet*(C) |
| Sulfonamide | *sul1* | - | *sul1* | - | - | *sul1,2* | - | *sulI* |
| Chloramphenicol | *ampK35* FS_aa29 | *cat42* | *ompK35* FS_aa29 | *catA2* | *ompK35* FS_aa134 | - | - | *catI* |
| Polymyxin | *ΔccrAB* | - | Δ*ccrAB* | - | - | - | - | - |
| | *mgrB* FS_aa8 | - | - | - | - | - | - | - |
| Macrolide | - | - | - | - | - | - | - | *mphA, mrx* |

The genome sequence of *K. pneumoniae* ST11 strain TK1401 was compared with that of other *K. pneumoniae* ST11 strains (JM45, HS11286, and KP69) from China (Figure 1). ST11 isolates JM45 (GenBank accession number CP006656) and HS11286 (GenBank accession number CP003200) were used for comparison in addition to closely related KP69. Overall, the genome structure was conserved. Differences were due to SNPs and insertions and deletions of mobile genetic elements, including the prophage, integrative conjugative element (ICE), transposon (Tn), and insertion sequence.

HS11286 has two ICEs: ICE*Kpn*HS11286-1 and ICE*Kpn*HS11286-2 [NPL 19]. ICE*Kpn*HS11286-2 was conserved in JM45, HS11286, KP69, and TK1401. By contrast, ICE*Kpn*HS11286-1, which was classified recently as ICE*Kp3*, was conserved in HS11286, KP69, and TK1401 (Figure 1). These data suggest that TK1401 belongs to ST11 strains have both of these ICEs, which may contribute to the pathogenicity of ST11 strains.

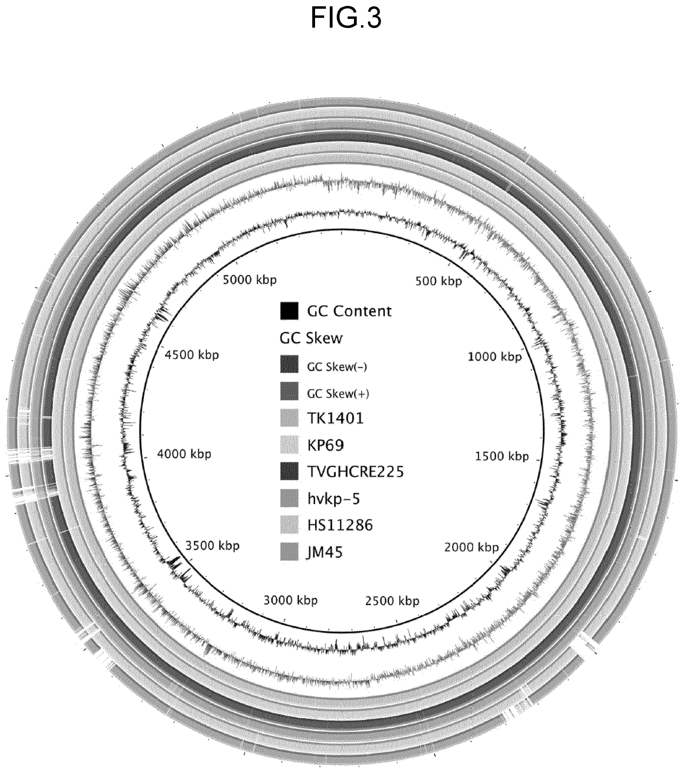

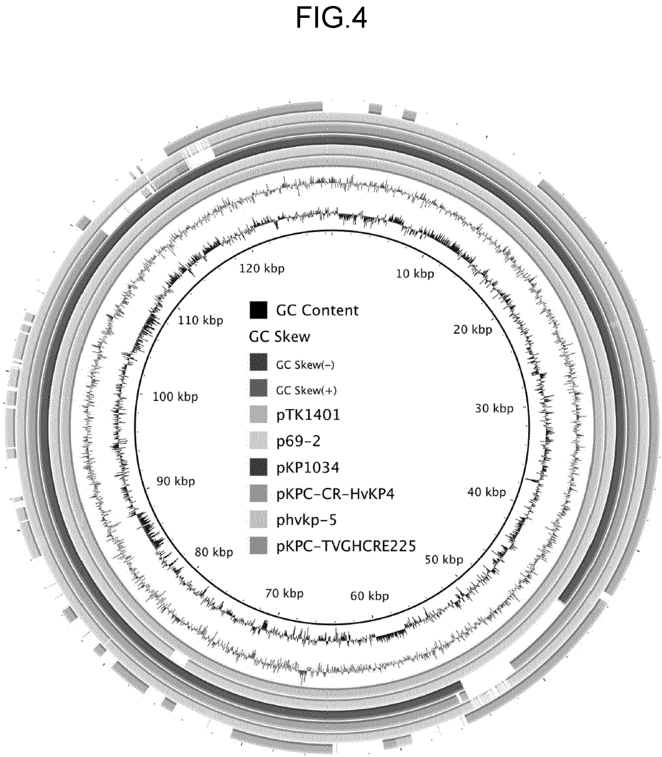

Comparing the genomes of pan-resistant TK1401 and non-pan-resistant KP69 revealed a deletion in TK1401 (Figure 3 - 5). The deleted region, which lies upstream of the IS*Kpn*25 transposase, contains genes encoding DinI, LtrA (a group II intron reverse transcriptase), DNA adenine methylase, DNA replication endonuclease, and eight hypothetical proteins and three prophage-related proteins. KP69 and TK1401 have eight and seven *ltrA* genes, respectively.

Most of the proteins, including DNA adenine methylase and DNA replication endonuclease, are thought to relate to prophage function. Dinl regulates recombination; loss of Dinl might upregulate recombination. CYD98_21555, one hypothetical protein, is similar to TraR, which belongs to the DskA_TraR superfamily. TraR is a global transcriptional regulator [NPL 21,22].

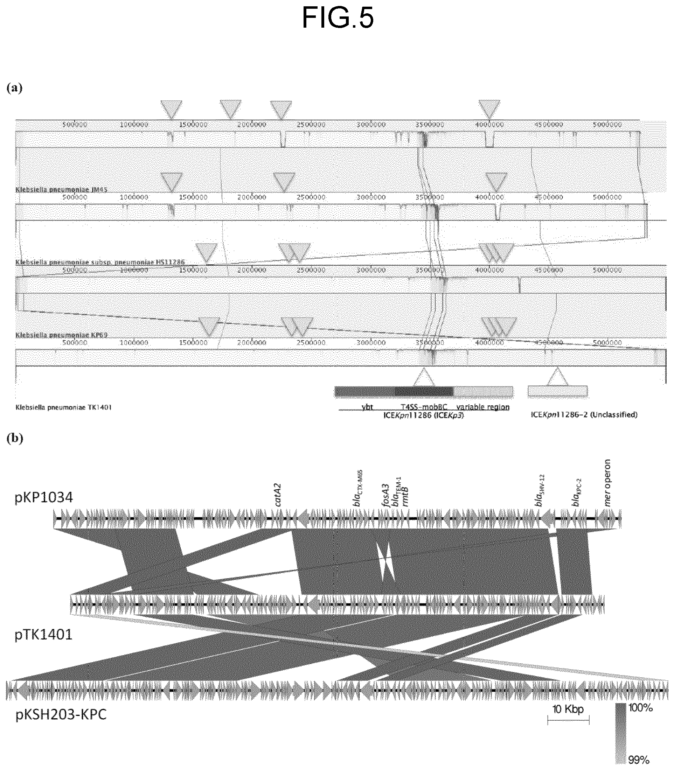

To find the IncR-F33:A-:B- plasmids having 7 resistance genes *fosA3, bla*_{KPC-2}, *bla*_{CTX-M-65}, *bla*_{SHV-12}, *bla*_{TEM-1}, *rmtB,* and *catA2,* NCBI BLASTN 2.10.0 were used by searching nucleotide collection (nr/nt). *rmtB, bla*_{KPC-2}, *bla*_{SHV-12}, *fosA3,* and *catA2* sequences used as genetic markers revealed three GenBank data, accession number KP893385, CP034324, LR59607). All three strains having each plasmid belong to ST11. These data suggest that genetic markers *(rmtB, bla*_{KPC-2}, *bla*_{SHV-12}, *fosA3,* and *catA2*) may be useful to find the multidrug-resistant IncR-F33:A-:B- plasmids, especially having 7 resistance genes fosA3, *bla*_{KPC-2}, *bla*_{CTX-M-65}, *bla*_{SHV-12}, *bla*_{TEM-1}, *rmtB,* and *catA2,* and understand its evolution in *K pneumoniae* ST11. *fosA3* gene was detected in *K. pneumoniae* ST11 having *wzi209* (K47) and *wzi64* (K67) type cps genes (NPL 18), suggesting *fosA3* plasmids might evolve in K47 and K67 type *K. pneumoniae* ST11. pKP1034, pTK1401, and pKSH203-KPC were compared using Easyfig (Figure 5(b)). This comparison suggested that pKP1034-like plasmids and the rearrangement by recombination might be caused by transposable elements (IS and Tn).

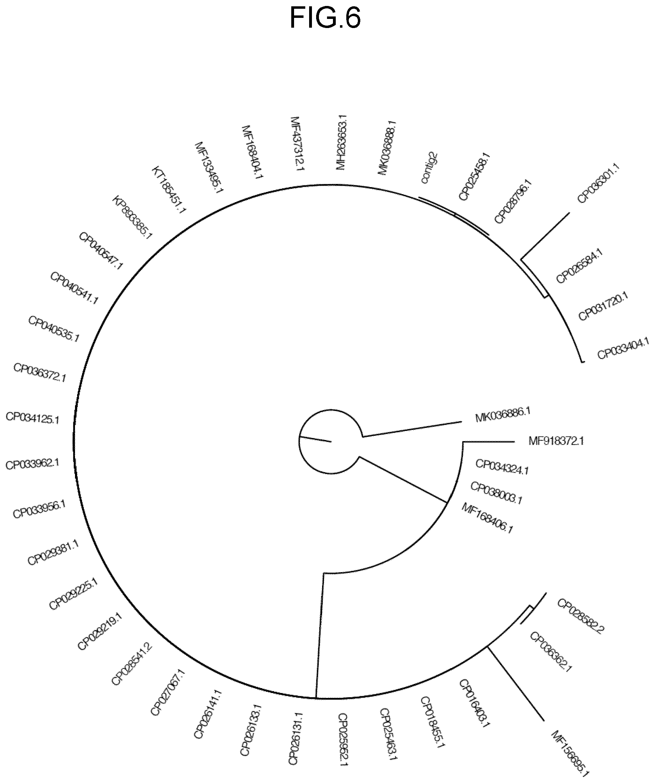

Plasmid pTK1401 identified from TK1401 genome sequence was phylogenetically analyzed with 41 of the top 97 BLAST hits for *K. pneumoniae* isolates (Figure 6). It was most similar to plasmid p69-2 from KPC-2-producing *K. pneumoniae* isolate KP69 (GenBank accession number CP025458) (99% query coverage, 99% identity). p69-2 was highly similar to the IncR-F33:A-:B- plasmid pKP1034 (GenBank accession number KP893385) having *fosA3, bla*_{KPC-2}, *bla*_{CTX-M-65}, *bla*_{SHV-12}, and *rmtB* from the epidemic KPC-2-producing *K. pneumoniae* isolate KP1034.

Next, we compared closely related phylogenetic group KPC-2-encoding plasmids, pKP1034, p69-2, and pTK1401, using Easyfig (Figure 2(b)). The comparison suggested that a pKP1034-like plasmid might have undergone rearrangement by recombination to form p69-2; further recombination of p69-2 might have led to the formation of pTK1401.

The IncR-F33:A-:B- plasmid having *fosA3, bla*_{KPC-2}, *bla*_{CTX-M-65}, *bla*_{SHV-12}, and *rmtB* formed a cluster with pKP1034, p69-2, pKPGD4, pKPC-CR-HvKP4, and pTK1401. These plasmids were identified from *K. pneumoniae* ST11 having a K47-type cps gene. pKPC-CR184 HvKP4 was isolated (and sequenced) from a hypervirulent KPC-2-producing *K*. *pneumoniae* ST11 strain having virulence plasmid pVir-CR-HvKP4 (NPL 23). In the present example, pTK1401 was identified from pan-resistant *K. pneumoniae* ST11 having a K47-type cps gene, which lacks virulence plasmids according to genomic analysis using BIGSdb. Further, major virulence genes were conserved in K47 ST11 isolates (Table 3).

**[Table 3]**

| Virulence gene | Allele number | | | | | |
|---|---|---|---|---|---|---|
| | *K. pneumoniae* | strain | | | | |
| | hvkp-5 | TVGHCRE225 | TK1401 | KP69 | HS11286 | JM45 |
| *fyuA* | **11** | **11** | **11** | **11** | **11** | - |
| *iroB*^{P} | - | 1 | - | - | - | - |
| *iroC*^{P} | - | U | - | - | - | - |
| *iroD*^{P} | - | 1 | - | - | - | - |
| *iroE*^{P} | - | U | - | - | - | - |
| *iroN*^{P} | - | 1 | - | - | - | - |
| *irp1* | - | **65** | **65** | **65** | 19 | - |
| *irp2* | **60** | **60** | **60** | **60** | 22 | - |
| *iucA*^{P} | **1** | 1 | - | - | - | - |
| *iucB*^{P} | **1** | 1 | - | - | - | - |
| *iucC*^{P} | **1** | U | - | - | - | - |
| *iucD*^{P} | **1** | 1 | - | - | - | - |
| *iucA*^{P} | **1** | 1 | - | - | - | - |
| *mrkA* | **2** | **2** | **2** | **2** | **2** | **2** |
| *mrkB* | **2** | **2** | **2** | **2** | **2** | **2** |
| *mrkC* | **2** | **2** | **2** | **2** | **2** | **2** |
| *mrkD* | **12** | **12** | **12** | **12** | **12** | **12** |
| *mrkF* | **8** | **8** | **8** | **8** | **8** | **8** |
| *mrkH* | **7** | **7** | **7** | **7** | **7** | **7** |
| *mrkI* | **15** | **15** | **15** | **15** | **15** | **15** |
| *mrkJ* | **12** | **12** | **12** | **12** | **12** | **12** |
| *rmpA2*^{P} | 9 | U | - | - | - | - |
| *rmpA*^{P} | - | U | - | - | - | - |
| *ybtA* | **1** | **1** | **1** | **1** | 9 | - |
| *ybtE* | **5** | **5** | **5** | **5** | 11 | - |
| *ybtP* | **5** | **5** | **5** | **5** | **5** | - |
| *ybtQ* | **6** | **6** | **6** | **6** | **14** | - |
| *ybtS* | - | - | - | - | **14** | - |
| *ybtT* | **5** | **5** | **5** | **5** | **5** | - |
| *ybtU* | **9** | **9** | **9** | **9** | **10** | - |
| *ybtX* | **11** | **11** | **11** | **11** | **11** | - |

| | | | | | | |
|---|---|---|---|---|---|---|
| P, plasmid-derived gene; U, undefined allele. The allele number of virulence genes was assigned by BIGSdb. Conserved alleles are shown in bold. | | | | | | |

TK1401 could be related to a highly pathogenic carbapenem-resistant *Klebsiella pneumoniae* identified recently in China and Taiwan (hvpk-5 GenBank accession number NJPJ00000000 and TVGHCRE225 GenBank accession number CP023722); however, it lacks a virulence plasmid (NPL 23,24).

The resistance genes on the plasmid and chromosome of the TK1401 isolate are listed in Table 1. Mutations in the quinolone-resistant determinant region of the chromosomal *gyrA* (D87G) and *parC* (S801) genes account for resistance to fluoroquinolones (NPL 28). A mutation in the *ramR* (T1621) transcriptional repressor gene confers resistance to tigecycline (NPL 27). Subsequent overexpression of the AcrAB efflux pump might contribute to resistance to multiple drugs, including chloramphenicol, fluoroquinolones, tetracyclines, and glycylcycline. We found that the colistin resistance of TK1401 is associated with a frameshift mutation in *mgrB* (FS_aa8).

The plasmid has carbapenemase gene *bla*_{KPC-2}, the ESBL gene *bla*_{CTX-M-65}, and β-lactamase genes *bla*_{TEM-1} and *bla*_{SHV-12}. *bla*_{SHV-11}. Five β-lactamases accounted for resistance to all of the β-lactam antibiotics listed in Table 1. In addition to β-lactamases, we detected chromosomal genes encoding outer membrane porins (*ompK35, ompK36,* and *ompK37*), which are associated with membrane permeability and drug resistance.

TK1401 has a frameshift mutation in *ompK35* (FS_aa29), which is different from that in *ompK35* (FS_aa134) has by KPC-producing *K. pneumoniae* ST11 strain HS11286 (NPL 25). The *ompK36* gene in TK1401 is identical to an *ompK36* variant harbored by KPC-producing *K. pneumoniae* ST11 strain HS092187 isolated in China (GenBank accession number JX310552), and is associated with carbapenem resistance [NPL 26]. The *ompK37* gene of TK1401 is identical to that of HS11286 and NDM-producing *K*. *pneumoniae* ST11 isolate JIE2713 (GenBank accession number KC534871) [NPL 26]. All *ompK* genes in TK1401 are conserved in KP69, suggesting that KP69 is a possible ancestral strain. The present inventors found that the colistin resistance of a pan-resistant *K. pneumoniae* is associated with a frameshift mutation in *mgrB* (FS_aa8). Colistin-resistant ST11 strains and KPC-2- producing ST11 strains have been reported in Asia. The emergence of *K. pneumoniae* ST11 having both phenotypes is a threat to both clinical therapy and infection control.

As described above, by using the complete genome sequence of the *K*. *pneumoniae* isolate TK1401 analyzed by the inventors, the sequence of the complete genome of the bacteria in the sample can be compared with the nucleotide sequence of SEQ ID NO: 1 and 2, and based on the rate of agreement, it can be determined whether the bacteria in the sample are pan-resistant *K. pneumoniae* or not. In addition, effective antimicrobial agents can be screened by supplying samples containing candidate antimicrobial agents against pan-resistant *K. pneumoniae* found by the inventors.

The strains and plasmids used in the phylogenetic tree analysis of *K. pneumoniae* are shown in Tables 4 - 7.

**[Table 4]**

| GenBank accession number. Strain name |
|---|
| CP006659.2 Klebsiella pneumoniae strain ATCC BAA-2146 chromosome 1, complete sequence |
| CP006918.1 Klebsiella pneumoniae 30684/NJST258_2, complete genome |
| CP007727.1 Klebsiella pneumoniae subsp. pneumoniae KPNTH10, complete genome |
| CP008827.1 Klebsiella pneumoniae subsp. pneumoniae KPNTH1, complete genome |
| CP00SR31.1 Klebsiella pneumoniae subsp. pneumoniae KPR0928, complete sequence |
| CP009771.1 Klebsiella pneumoniae subsp. pneumoniae strain KPNIH33, complete genome |
| CP009775.1 Klebsiella pnemnoniae subsp. pneumoniae strain KPNIH32, complete genome |
| CP010361.1 Klebsiella pneumoniae strain 32192, complete genome |
| CP010392.1 Klebsiella pneumoniae strain 34618, complete genome |
| CP011647.1 Klebsiella pneumoniae strain CAV1596, complete genome |
| CP011976.1 Klebsiella pneumoniae DMC1097, complete genome |
| CP011980.1 Klebsiella pneumoniae 500_1420, complete genome |
| CP011985.1 Klebsiella pneumoniae UHKPC07, complete genome |
| CP011989.1 Klebsiella pneumoniae UHKPC33, complete genome |
| CP014294.1 Klebsiella pneumoniae strain KP38731, complete genome |
| CP014647.1 Klebsiella pneumoniae strain KPNIH36, complete genome |
| CP015392.1 Klebsiella pneumoniae strain CR14, complete genome |
| CP015822.1 Klebsiella pneumoniae isolate blood sample 2 chromosome, complete genome |
| CP018352.1 Klebsiella pneumoniae strain CAV1417 chromosome, complete genome |
| CP0138336.1 Klebsiella pneumoniae strain CAV1453 chromosome, complete genome |
| CP018427.1 Klebsiella pneumoniae strain MNCRE69 chromosome, complete genome |
| CP018428.1 Klebsiella pneumoniae strain MNCRE78 chromosome, complete genome |
| CP018437.1 Klebsiella pneumoniae strain MNCRE53 chromosome, complete genome |
| CP018438.1 Klebsiella pneumoniae strain Kp_Goe_822917, complete sequence |
| CP018454.1 Klebsiella pneumoniae strain SWU01 chromosome, complete genome |
| CP018692.1 Klebsiella pneumoniae strain Kp_Goe_82158S, complete sequence |
| CP018916.1 Klebsiella pneumoniae strain AR_0049, complete genome |
| CP018883.1 Klebsiella pneumoniae subsp. pneumoniae strain BR7 chromosome, complete genome |
| CP018885.1 Klebsiella pneumoniae subsp. pneumoniae strain BR21 chromosome, complete genome |
| CP019772.1 Klebsiella pneumoniae subsp. pneumoniae strain KPN_KPC_HUG_07 genome |
| CP020071.1 Klebsiella pneumoniae strain AR_0115, complete genome |
| CP020S37.1 Klebsiella pneumoniae strain BK13043 chromosome, complete genome |
| CP020901.1 Klebsiella pneumoniae strain K66-45 chromosome, complete genome |
| CP021549.1 Klebsiella pneumoniae strain AR_0112, complete genome |
| CP021685.1 Klebsiella pneumoniae strain AR_0146, complete genome |
| CP021718.1 Klebsiella pneumoniae strain AR_0129, complete genome |
| CP021751.1 Klebsiella pneumoniae strain AR_0113 chromosome, complete genome |
| CP021833.1 Klebsiella pneumoniae strain AR_0120, complete genome |
| CP021859.1 Klebsiella pneumoniae strain AR_0125 chromosome, complete genome |
| CP021950.1 Klebsiella pneumoniae strain AR_0148 chromosome, complete genome |
| CP022143.1 Klebsiella pneumoniae 704SK6 genome |
| CP022373.1 Klebsiella pneumoniae strain BIC-1 chromosome, complete genome |
| CP022691.1 Klebsiella pneumoniae subsp. pneumoniae strain AUSMDU00008079 chromosome, complete genome |
| CP022882.1 Klebsiella pneumoniae strain 911021 chromosome, complete genome |
| CP022997.1 Klebsiella pneumoniae strain 721005 chromosome, complete genome |
| CP023722.1 Klebsiella pneumoniae strain TVGHCRE225 chromosome, complete genome |
| CP023907.1 Klebsiella pneumoniae strain FDAARGOS_436 chromosome, complete genome |
| CP023933.1 Klebsiella pneumoniae strain FDAARGOS_443 chromosome, complete genome |
| CP023941.1 Klebsiella pneumoniae strain FDAARGOS_444 chromosome, complete genome |
| CP025005.1 Klebsiella pneumoniae strain AUSMDU00003562 chromosome, complete genome |
| CP025008.1 Klebsiella pneumoniae strain AUSMDU00008119 chromosome, complete genome |

**[Table 5]**

| |
|---|
| CPD23037.1 Klebsiella pneumoniae strain NU-CRE047 chromosome, complete genome |
| CP025456.1 Klebsiella pneumoniae strain KP69 chromosome, complete genome |
| CP025951.1 Klebsiella pneumoniae subsp. pneumoniae strain GD4 chromosome, complete genome |
| CP026130.1 Klebsiella pneumoniae strain F1 chromosome, complete genome |
| CP016131.1 Klebsiella pneumoniae strain F5 chromosome, complete genome |
| CP026140.1 Klebsiella pneumoniae strain F127 chromosome, complete genome |
| CP026145.1 Klebsiella pneumoniae strain F132 chromosome, complete genome |
| CP026149.1 Klebsiella pneumoniae strain F138 chromosome, complete genome |
| CP026155.1 Klebsiella pneumoniae strain B12(AN) chromosome, complete genome |
| CP026585.3 Klebsiella pneumoniae strain WCHKP649 chromosome, complete genome |
| CP027036.1 Klebsiella pneumoniae strain 16_GR_13 chromosome, complete genome |
| CP027053.1 Klebsiella pneumoniae strain 2_GR_12 chromosome, complete genome |
| CP027068.3 Klebsiella pneumoniae strain WCHKPSF4 chromosome, complete genome |
| CP027146.1 Klebsiella pneumoniae strain AR_0363 chromosome, complete genome |
| CP027160.1 Klebsiella pneumoniae strain AR_0361 chromosome, complete genome |
| CP027697.1 Klebsiella pneumoniae strain KP30835 chromosome, complete genome |
| CP028180.1 Klebsiella pneumoniae strain CFSANDS4110 chromosome, complete genome |
| CP028542.3 Klebsiella pneumoniae strain WCHKP2 chromosome, complete genome |
| CP028783.2 Klebsiella pneumoniae subsp. pneumoniae strain SCKP020046 chromosome, complete genome |
| CP028793.2 Klebsiella pneumoniae strain WCHKP020030 chromosome, complete genome |
| CP028797.2 Klebsiella pneumoniae strain WCHKP040035 chromosome, complete genome |
| CP028306.2 Klebsiella pneumoniae strain WCHKP7E2 chromosome, complete genome |
| CPO28994.1 Klebsiella pneumoniae strain AR_0079 chromosome, complete genome |
| CP029099.1 Klebsiella pneumoniae strain AR438 chromosome, complete genome |
| CP029216.1 Klebsiella pneumoniae strain L201 chromosome |
| CP029220.1 Klebsiella pneumoniae strain L388 chromosome |
| CP029226.1 Klebsiella pneumoniae strain L491 chromosome |
| CP029384.2 Klebsiella pneumoniae subsp. pneumoniae strain SCKP020079 chromosome, complete genome |
| CP029689.1 Klebsiella pneumoniae strain 160111 chromosome, complete genome |
| CP030341.1 Klebsiella pneumoniae strain AR_362 chromosome, complete genome |
| CP031721.1 Klebsiella pneumoniae strain WCHKP3 chromosome, complete genome |
| CP032163.1 Klebsiella pneumoniae strain XJ-K1 chromosome, complete genome |
| CP032207.1 Klebsiella pneumoniae strain AR_0109 chromosome, complete genome |
| CP033242.1 Klebsiella pneumoniae strain 675920 chromosome 675920 |
| CP033954.1 Klebsiella pneumoniae strain L39_2 chromosome, complete genome |
| CP033960.1 Klebsiella pneumoniae strain L482 chromosome, complete genome |
| CP034249.1 Klebsiella pneumoniae strain KP18-29 chromosome, complete genome |
| CP034327.1 Klebsiella pneumoniae isolate KSH203 chromosome, complete genome |
| CP034760.1 Klebsiella pneumoniae strain NB5306 chromosome, complete genome |
| CP035540.1 Klebsiella pneumoniae subsp. pneumoniae strain CCRI-22199 chromosome, complete genome |
| CP036300.1 Klebsiella pneumoniae subsp. pneumoniae strain WCHKP015093 chromosome, complete genome |
| CP036305.1 Klebsiella pneumoniae strain WCHKP020098 chromosome, complete genome |
| CP036371.1 Klebsiella pneumoniae strain WCHKP020037 chromosome, complete genome |
| CP038002.1 Klebsiella pneumoniae strain SCKP020009 chromosome, complete genome |
| CP040539.1 Klebsiella pneumoniae strain CR-HvKP4 chromosome, complete genome |
| CP040545.1 Klebsiella pneumoniae strain CR-HvKP5 chromosome, complete genome |
| CP043047.1 Klebsiella pneumoniae strain KLP268 chromosome, complete genome |
| LR130548.1 Klebsiella pneumoniae strain KPC2 genome assembly, chromosome: 1 |
| LT216436.1 Klebsiella pneumoniae isolate 207M1D0-sc-2013-04-03T11:21:06Z-1606409 genome assembly, chromosome: 1 |
| contig1 Klebsiella pneumoniae strain TK1401 chromosome, complete genome |

**[Table 6]**

| GenBank accession number. Plasmid name |
|---|
| AP018136.1 Escherichia coli plasmid pM105_FII DNA, complete genome, isolate: M105 |
| AP018138.1 Escherichia coli plasmid pM107_FII DNA, complete genome, isolate: M107 |
| AP018144.1 Escherichia coli plasmid pM214_FII DNA, complete genome, isolate: M214 |
| AP018572.1 Escherichia coli MH13-051M plasmid pMH13-051M_1 DNA, complete genome |
| CP016403.1 Klebsiella pneumoniae subsp. pneumoniae strain F5 plasmid pF5, complete sequence |
| CP017086.1 Proteus mirabilis strain T18 plasmid pT18, complete sequence |
| CP017287.1 Klebsiella variicola strain GJ3 plasmid pKPGJ-3c, complete sequence |
| CP017852.1 Klebsiella variicola strain GJ2 plasmid pKPGJ-2c, complete sequence |
| CP018435.1 Klebsiella pneumoniae strain SWU01 plasmid unnamed, complete sequence |
| CP021195.1 Escherichia coli strain H17 plasmid pH17-2, complete sequence |
| CP021210.1 Escherichia coli strain strain Z247 plasmid p2474-NDM1, complete sequence |
| CP023895.1 Escherichia coli strain FDAARGOS_433 plasmid unnamed2, complete sequence |
| CP023934.1 Klebsiella pneumoniae strain FDAARGOS_443 plasmid unnamed3, complete sequence |
| CP023942.1 Klebsiella pneumoniae strain FDAARGOS_444 plasmid unnamed2 |
| CP024430.1 Klebsiella pneumoniae strain DA48896 plasmid p48896_1, complete sequence |
| CP024836.1 Klebsiella pneumoniae strain CRKP-2297 plasmid pCRKP-2297_2, complete sequence |
| CP024840.1 Klebsiella pneumoniae strain CRKP-1215 plasmid pCRKP-1215_2, complete sequence |
| CP025458.1 Klebsiella pneumoniae strain KP69 plasmid p69-2, complete sequence |
| CP025463.1 Klebsiella pneumoniae strain F44 plasmid p44-2, complete sequence |
| CP025468.1 Klebsiella pneumoniae strain JS187 plasmid p187-2, complete sequence |
| CP025952.1 Klebsiella pneumoniae subsp. pneumoniae strain GD4 plasmid pKPGD4, complete sequence |
| CP026131.1 Klebsiella pneumoniae strain F1 plasmid pF1_1, complete sequence |
| CP026133.1 Klebsiella pneumoniae strain F5 plasmid pF5_1, complete sequence |
| CP026141.1 Klebsiella pneumoniae strain F127 plasmid pF127_1, complete sequence |
| CP026147.1 Klebsiella pneumoniae strain F132 plasmid pF132_2, complete sequence |
| CP026152 1 Klebsiella pneumoniae strain F138 plasmid pF138_3, complete sequence |
| CP026584.1 Klebsiella pneumoniae strain WCHKP649 plasmid pKPC2 _095649, complete sequence |
| CP026589.1 Klebsiella pneumoniae strain NUHL30457 plasmid p3, complete sequence |
| CP027067.1 Klebsiella pneumoniae strain WCHKP8F4 plasmid pKPC2_095084, complete sequence |
| CP028541.2 Klebsiella pneumoniae strain WCHKP2 plasmid pKPC2_020002, complete sequence |
| CP028382.2 Klebsiella pneumoniae strain WCHKP36 plasmid pKPC2_020036, complete sequence |
| CP028790.2 Klebsiella pneumoniae strain WCHKP020030 plasmid pKPC2_020030, complete sequence |
| CP028796.1 Klebsiella pneumoniae strain WCHKP040035 plasmid pKPC2_040035, complete sequence |
| CP028805.1 Klebsiella pneumoniae strain WCHKP7E2 plasmid pKPC2_085072, complete sequence |
| CP029219.1 Klebsiella pneumoniae strain L201 plasmid pKPC-1201 |
| CP029225.1 Klebsiella pneumoniae strain L388 plasmid pKPC-1.388 |
| CP029230.1 Klebsiella pneumoniae strain L491 plasmid pKPC -L491 |
| CP029381.1 Klebsiella pneumoniae subsp. pneumoniae strain SCKP020079 plasmid pKPC2_020079, complete sequence |
| CP030134 1 Klebsiella pneumoniae strain 160111 plasmid pKPC2_L111, complete sequence |
| CP031720.1 Klebsiella pneumoniae strain WCHKP3 plasmid pKPC2_020003, complete sequence |
| CP032166.1 Klebsiella pneumoniae strain XJ-K1 plasmid unnamed3, complete sequence |
| CP033395.1 Klebsiella pneumoniae strain WCHKP015625 plasmid _015625, complete sequence |
| CP033404.1 Klebsiella pneumoniae strain WCHKP115069 plasmid pKPC2_115069, complete sequence |
| CP033956.1 Klebsiella pneumoniae strain L39_2 plasmid p3_L39, complete sequence |
| CP033962.1 Klebsiella pneumoniae strain L482 plasmid p3 L382, complete sequence |
| CP034125.1 Klebsiella pneumoniae strain BJCFK909 plasmid p2b2, complete sequence |
| CP034324.1 Klebsiella pneumoniae isolate KSH203 plasmid pKSH203-KPC, complete sequence |
| CP034736.1 Escherichia coli strain L53 plasmid pL53-3 |
| CP034846.1 Escherichia coli strain L103-2 plasmid p103-2-4 |
| CP036301.1 Klebsiella pneumoniae subsp. pneumoniae strain WCHKP015093 plasmid pKPC2_015093, complete sequence |
| CP030306.1 Klebsiella pneumoniae strain WCHKP020098 plasmid pKPC2_020098, complete sequence |
| CP036362.1 Klebsiella pneumoniae strain WCHKP2080 plasmid pKPC2_095080, complete sequence |
| CP036361.1 Klebsiella pneumoniae strain WCHKP115068 plasmid pKPC12_115068, complete sequence |
| CP336372.1 Klebsiella pneumoniae strain WCHKP020037 plasmid pKPC2_020037, complete sequence |
| CP03S003.1 Klebsiella pneumoniae strain SCKP020009 plasmid pKPC2_020009, complete sequence |

**[Table 7]**

| |
|---|
| CP040180.1 Klebsiella pneumoniae strain LSH-KPN25 plasmid pLSH-KPN25-1, complete sequence |
| CP040535.1 Klebsiella pneumoniae strain CR-HvKP1 plasmid pCR-HvKP1-KPC, complete sequence |
| CP040541.1 Klebsiella pneumoniae strain CR-HvKP4 plasmid pCR-HvKP4-pKPC, complete sequence |
| CP040547.1 Klebsiella pneumoniae strain CR-HvKP5 plasmid pCR-HvKP5-KPC, complete sequence |
| CP040807.1 Escherichia fergusonii strain EFCF056 plasmid pEF02, complete sequence |
| CP042601.1 Escherichia coli strain NCYU-29-69 plasmid pNCYU-29-69-2, complete sequence |
| FJ628167.2 Klebsiella pneumoniae strain KP048 plasmid pKP048, complete sequence |
| JF927996.1 Escherichia coli plasmid pXZ, complete sequence |
| JN232517.1 Escherichia coli strain 7A8 plasmid pHN7A8, complete sequence |
| JQ432559.1 Escherichia coli plasmid pHK23a. complete sequence |
| JX997935.2 Escherichia coli strain 3A11 plasmid pHK3A11, complete sequence |
| KPS93385.1 Klebsiella pneumoniae subsp. pneumoniae strain KP1034 plasmid pKP1034, complete sequence |
| KR078259.1 Escherichia coli strain YD472 plasmid pYHCC, complete sequence |
| KT185451.1 Klebsiella pneumoniae plasmid pCT-KPC strain LJ04, complete sequence |
| KT725788.1 Klebsiella pneumoniae strain ST147 plasmid pCC1410-1, complete sequence |
| KT725789.1 Klebsiella pneumoniae strain ST147 plasmid pCC1409-1, complete sequence |
| KX503323.1 Escherichia coli strain HNEC46 plasmid PHNEC46, complete sequence |
| KY130431.1 Klebsiella pneumoniae plasmid pABC143C-NDM, complete sequence |
| LN897474.2 Klebsiella pneumoniae p397Kp plasmid, complete sequence |
| LN897475.2 Klebsiella pneumoniae p477Kp plasmid, complete sequence |
| LR697130.1 Escherichia coli isolate 8395d878-b38d-11e9-8998-68b599768938 genome assembly, plasmid: p14ARS_NMC0074-3 |
| LS992188.1 Escherichia coli isolate Escherichia coli str. 3426 genome assembly, plasmid: 4 |
| LT968717.1 Klebsiella pneumoniae genome assembly, plasmid: 31 |
| LT968753.1 Klebsiella pneumoniae genome assembly, plasmid: 67 |
| MF133495.1 Klebsiella pneumoniae strain 675920 plasmid p675920-1, complete sequence |
| MF156695.1 Klebsiella pneumoniae strain 1642 plasmid p1642-1, complete sequence |
| MF168402.1 Klebsiella pneumoniae strain 1068 plasmid p1068-KPC, complete sequence |
| MF168403.1 Klebsiella pneumoniae strain 12139 plasmid p12139-KPC, complete sequence |
| MF168404.1 Klebsiella pneumoniae strain 20049 plasmid p20049-KPC, complete sequence |
| MF168405.1 Klebsiella pneumoniae strain 64917 plasmid p64917-KPC. complete sequence |
| MF168406.1 Klebsiella pneumoniae strain 283747 plasmid p283747-KPC, complete sequence |
| MF437312.1 Klebsiella pneumoniae strain CR-HvKP4 plasmid pKPC-CR-HvKP4, complete sequence |
| MF918372.1 Klebsiella pneumoniae plasmid p1512-KPC, complete sequence |
| MG591701.1 Escherichia coli strain EC36 plasmid pEC 36-2, complete sequence |
| MG764548.1 Escherichia coli strain 11011 plasmid p11011-fosA, complete sequence |
| MH255829.1 Klebsiella pneumoniae subsp. pneumoniae strain SH9 plasmid pSH9-CTX-TEM, complete sequence |
| MH263653.1 Klebsiella pneumoniae strain QL24 plasmid pKPC-QL24, complete sequence |
| MEK036886.1 Klebsiella pneumoniae strain 283149 plasmid p283149-KPC, complete sequence |
| MK036887.1 Klebsiella pneumoniae strain 397108 plasmid p397108-KPC, complete sequence |
| MK036888.1 Klebsiella pneumoniae strain 911021 plasmid p911021-KPC, complete sequence |
| MK396843.1 Klebsiella pneumoniae strain LSH-KPN148 plasmid pLSH-KPN148-1, complete sequence |
| MK433206.1 Klebsiella pneumoniae subsp. pneumoniae K15 plasmid pK15-FOS, complete sequence |
| contig2 Klebsiella pneumoniae strain TK1401 plasmid pTK1401, complete sequence |

[Sequence Listing]

## Claims

1. A detection method for pan-resistant *K. pneumoniae* comprising of:
performing complete genome analysis of the bacteria contained in the sample; and
comparing the nucleotide sequence contained in the obtained complete genome with nucleotide sequences of SEQ ID NO: 1 and 2, and determining whether or not the bacteria in the sample is pan-resistant *K. pneumoniae* based on the rate of agreement, wherein when the rate of agreement is 99.9% or higher the bacteria in the sample is pan-resistant *K. pneumoniae.*

2. A screening method for antimicrobial agents comprising of:
supplying a sample containing a candidate antimicrobial agent to pan-resistant *K*. *pneumoniae* which has chromosomal DNA comprising of the nucleotide sequence of SEQ ID NO: 1 and plasmid DNA comprising of the nucleotide sequence of SEQ ID NO: 2.

3. A recording media and database **characterized by** the storage of nucleotide sequence information of SEQ ID NO: 1 and 2 and comprising instructions that when executed on a computer perform the computer implemented steps of claim 1.

## Patentansprüche

1. Nachweisverfahren für pan-resistente *K. pneumoniae,* umfassend:
Durchführen einer Vollgenomanalyse der in der Probe enthaltenen Bakterien; und
Vergleichen der in dem erhaltenen vollständigen Genom enthaltenen Nukleotidsequenz mit den Nukleotidsequenzen von SEQ ID NO: 1 und 2 und Bestimmen, ob die Bakterien in der Probe pan-resistente *K. pneumoniae* sind oder nicht, basierend auf der Übereinstimmungsrate, wobei die Bakterien in der Probe pan-resistente *K. pneumoniae* sind, wenn die Übereinstimmungsrate 99,9 % oder höher ist.

2. Screening-Verfahren für antimikrobielle Wirkstoffe, umfassend:
Zuführen einer Probe, die einen antimikrobiellen Wirkstoffkandidaten enthält, zu pan-resistenten *K. pneumoniae,* die chromosomale DNA, umfassend die Nukleotidsequenz von SEQ ID NO: 1 und Plasmid-DNA, umfassend die Nukleotidsequenz von SEQ ID NO: 2, aufweist.

3. Aufzeichnungsmedium und Datenbank, die durch die Speicherung von Nukleotidsequenzinformation von SEQ ID NO: 1 und 2 gekennzeichnet sind und Anweisungen umfassen, die, wenn sie auf einem Computer ausgeführt werden, die computerimplementierten Schritte des Anspruchs 1 ausführen.

## Revendications

1. Procédé de détection de K. *pneumoniae* pan-résistante comprenant les étapes consistant à :
effectuer une analyse du génome complet de la bactérie contenue dans l'échantillon ; et
comparer la séquence nucléotidique contenue dans le génome complet obtenu avec des séquences nucléotidiques des SEQ ID NO : 1 et 2, et déterminer si oui ou non la bactérie dans l'échantillon est K. *pneumoniae* pan-résistante sur la base du taux de concordance, dans lequel lorsque le taux de concordance est de 99,9 % ou plus, la bactérie dans l'échantillon est K. *pneumoniae* pan-résistante.

2. Procédé de criblage d'agents antimicrobiens comprenant l'étape consistant à :
fournir un échantillon contenant un agent antimicrobien candidat à K. *pneumoniae* pan-résistante qui présente un ADN chromosomique comprenant la séquence nucléotidique de SEQ ID NO : 1 et un ADN plasmidique comprenant la séquence nucléotidique de SEQ ID NO : 2.

3. Supports d'enregistrement et base de données **caractérisés par** le stockage d'informations de séquence nucléotidique de SEQ ID NO : 1 et 2 et comprenant des instructions qui, lorsqu'elles sont exécutées sur un ordinateur, effectuent les étapes mises en œuvre par ordinateur de la revendication 1.
